(19) Europäisches Patentamt
European Patent Office
Office européen des brevets

(11) Numéro de publication : **0 384 088 B1**

(12) # FASCICULE DE BREVET EUROPEEN

(45) Date de publication du fascicule du brevet : **31.03.93 Bulletin 93/13**

(51) Int. Cl.$^5$ : **C07C 217/48,** A61K 31/135

(21) Numéro de dépôt : **89401642.7**

(22) Date de dépôt : **13.06.89**

(54) **La (+) 1-[(3,4,5-triméthoxy) benzyloxyméthyl]-1-phényl-N,N-diméthyl-n-propylamine, son procédé de préparation et son application en thérapeutique.**

(30) Priorité : **20.02.89 FR 8902177**

(43) Date de publication de la demande : **29.08.90 Bulletin 90/35**

(45) Mention de la délivrance du brevet : **31.03.93 Bulletin 93/13**

(84) Etats contractants désignés : **AT BE CH DE ES FR GB GR IT LI LU NL SE**

(56) Documents cités : **GB-A- 2 054 588**

(73) Titulaire : **JOUVEINAL S.A.**
**Tour Maine Montparnasse 33 Avenue du Maine**
**F-75755 Paris Cedex 15 (FR)**

(72) Inventeur : **Aubard, Gilbert Gustave**
**7, Chemin de la Savetière**
**F-91120 Palaiseau (FR)**
Inventeur : **Calvet, Alain Pierre**
**Résidence Ronsard 3, rue Gatine**
**F-94240 L'Hay-les Roses (FR)**
Inventeur : **Grouhel, Agnès**
**2, rue des Peupliers**
**F-92190 Meudon (FR)**
Inventeur : **Jacobelli, Henri**
**65 Avenue du Général de Gaulle**
**F-91550 Paray-Vieille Poste (FR)**
Inventeur : **Junien, Jean-Louis**
**36 Avenue Eiffel**
**F-92310 Sevres (FR)**
Inventeur : **Pascaud, Xavier Bernard Louis**
**41, rue de Charenton**
**F-75012 Paris (FR)**
Inventeur : **Roman, François Joseph**
**10, rue de Dieppe**
**F-92400 Courbevoie (FR)**

(74) Mandataire : **Bourgognon, Jean-Marie et al**
**Cabinet Flechner 22, Avenue de Friedland**
**F-75008 Paris (FR)**

**EP 0 384 088 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un médicament qui, administré par voie orale, d'une part augmente la vidange gastrique, d'autre part est antispasmodique et, en conséquence, agit sur divers dysfonctionnements du tractus gastrointestinal.

Il est connu que la vidange gastrique peut être augmentée ou accélérée par divers composés. Ainsi, le métoclopramide qui est proposé depuis 1964, comme antiémétique et modificateur du comportement digestif possède cette propriété. Cependant, ce produit qui appartient à la classe des neuroleptiques est d'un emploi délicat tel que l'indiquent les contre-indications, précautions d'emploi, inter-actions médicamenteuses et effets indésirables cités au "Dictionnaire Vidal" - Ed. 1988 p. 1320.

Ainsi, les restrictions indiquées pour son utilisation associée aux dérivés anticholinergiques, aux composés antiparkinsoniens et aux neuroleptiques avec lesquels il est signalé soit des inhibitions d'activités soit des possibles synergies d'effet central limitent notablement son indication.

Récemment, M. Gué et Coll. (Gastroenterol. Clin. et Biol., 1988, 12, 2 bis) montrent que, par voie orale, deux composés connus jusqu'alors pour leur propriété analgésique, provoquent une augmentation de la vidange gastrique chez le chien. Les auteurs expliquent le phénomène par le fait que ces produits : le Tifluadom et le U-50,488 ont une affinité préférentielle pour les récepteurs opiacés de type kappa et, vraisemblablement, agissent localement sur ces récepteurs situés dans la muqueuse gastrique pour provoquer une augmentation de vidange.

Toutefois, ces composés sont connus principalement pour avoir une activité analgésique générale, de type opiacé, lorsqu'ils sont administrés par voie orale (Pharmaprojects - V & D Publications, Ltd - Mai et Août 1988).

Cette activité est annoncée ne pas provoquer d'effet de dépendance comme la morphine mais être accompagnée d'un phénomène de tolérance qui nécessite l'administration de quantités croissantes du produit pour obtenir l'effet souhaité.

Aussi ces composés, n'ont été utilisés à ce jour qu'au titre de réactifs pharmacologiques et sans applications connues à la thérapeutique humaine sous la forme de médicaments.

La présente invention vise un médicament agissant sur le tractus gastro-intestinal à la fois par une action antispasmodique et par un effet d'augmentation de la vidange gastrique, ces actions étant néanmoins sensiblement dénuées d'effet central et de phénomènes de dépendance ou d'accoutumance.

Le médicament est caractérisé par le fait qu'il contient, à titre de principe actif, l'énantiomère dextrogyre d'un amino éther oxyde : la (+) 1-[(3,4,5-triméthoxy) benzyloxyméthyl]-1-phényl- N,N-diméthyl-n-propylamine de formule

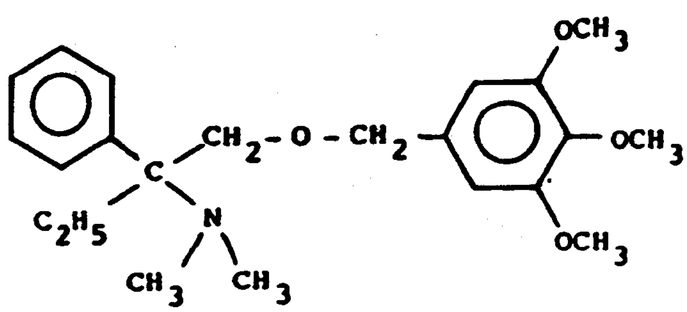

et ses sels d'addition avec les acides compatibles à une utilisation thérapeutique.

La présente invention a été réalisée de manière imprévue par une successsion d'études dont l'essentiel est relaté dans ce qui suit :

Au brevet des Etats unis d'Amérique n° 4,301,163 la demanderesse a revendiqué la protection d'amino éthers oxydes ayant de propriétés anesthésiques locales, spasmolytiques et analgésiques.

Afin de définir plus précisément cette dernière propriété pour le composé mentionné à l'exemple 2 du brevet, on a effectué la résolution optique de ce produit racémique et obtenu d'une part l'énantiomère dextrogyre qui est le produit de cette invention et, d'autre part l'énantiomère lévogyre.

Il est reconnu que la recherche "in vitro" de l'affinité de liaison aux récepteurs opiacés est un moyen d'étude des composés potentiellement analgésiques (Opiate receptor binding in Drug Research. Eric J. Simon dans "Receptor binding in Drug Research" p. 183-202 - Ed. Robert A. O'Brien-Dekker-1986).

Les composés optiquement actifs ainsi que ceux préférés au brevet des Etats-Unis d'Amérique, le produit nommé U 50,488 et la morphine à titre de référence ont été engagés dans cet essai selon la technique décrite par F. Roman et coll. dans J. Pharm. Pharmacol. 1987, 39, p. 404-407.

A l'issue de l'étude, on a constaté que le produit de l'invention se distingue des autres composés par le fait qu'il montre une affinité de liaison aux récepteurs mu, delta et kappa étudiés et ce à la différence des composés préférés du brevet des Etats Unis d'Amérique précité qui montrent surtout une affinité mu et du composé U 50,488 qui, tel qu'annoncé, se lie spécifiquement aux récepteurs kappa. Ces résultats sont significatifs d'activités analgésiques potentielles pour ces produits (CRC Handbook of Stereoisomers : Drugs in Psychopharmacology CRC Press, Inc - 1984 p. 402) ; ils ont donc été engagés dans un test "in vivo" reconnu approprié à déterminer cet effet.

D'une façon surprenante, le composé de l'invention, dans cet essai, est pratiquement dépourvu d'effet analgésique et ce contrairement aux autres produits qui se montrent actifs.

Une ultime tentative pour déterminer l'expression "in vivo" des propriétés constatées "in vitro" pour le produit a été alors réalisée par le test réalisé par M. Gué et coll. (Réf. déjà citée).

Mis à l'essai, le produit de l'invention montre sans équivoque une activité favorisant la vidange gastrique des solides chez le chien. En outre, l'effet observé est inhibé par des composés reconnus antagonistes opiacés.

Ainsi, d'une façon inattendu et particulière, le composé de l'invention qui montre "in vitro" une affinité aux récepteurs opiacés mu, delta et kappa, n'exerce pas "in vivo" et tel qu'on pourrait l'attendre une activité analgésique centrale mais une activité locale sur les récepteurs opiacés de la muqueuse gastrique et provoque ainsi l'augmentation de la vidange des solides chez le chien.

Par ailleurs, il est également remarquable que le produit possède aussi une activité antispasmodique.

Ainsi, en solution aqueuse, et à une concentration 1,70-$10^{-5}$ molaire le produit inhibe 50 % de l'amplitude des spasmes provoqués par le chlorure de baryum sur le duodénum de rat. Dans ce test qui est réalisé "in vitro" la morphine est inactive à une concentration plus de 5 fois supérieure à celle du produit de l'invention.

La dualité d'effets du produit s'exprimant par l'augmentation de la vidange gastrique des solides et l'abolition des spasmes intestinaux est particulièrement intéressante par l'étendue des actions sur les divers étages du tractus gastrointestinal.

De nombreux dysfonctionnements du tractus sont susceptibles d'être traités. On cite, entre autres exemples les troubles gastrointestinaux provoqués par des traitements médicamenteux de longue durée comme par certains analgésiques ou neuroleptiques.

Ainsi, le produit de l'invention est approprié à normaliser l'activité du tractus gastrointestinals chez les patients traités par des composés analgésiques de type morphinique qui sont connus ralentir la vidange gastrique et provoquer des spasmes de certains muscles intestinaux (Goodman and Gilman's "The Pharmacological Basis of Therapeutics" - 6ème ed. 1980 - p. 503 à 504).

Les essais et les résultats qui viennent d'être relatés et qui ont permis d'aboutir à l'invention sont développés de façon détaillée dans ce qui suit.

Les essais comparatifs qui ont permis de déterminer les propriétés particulières des produits de l'invention et plus particulièrement du D (-) tartrate de (+) 1-[(3,4,5-triméthoxy) benzyloxyméthyl]-1-phényl-N-N-diméthyl-n-propylamine qui est préféré ont été réalisés avec les composés racémiques du brevet des Etats-Unis d'Amérique n° 4,301,163 à savoir la (+/-)1-[(3,4-diméthoxy)benzyloxyméthyl]-1-phényl-N,N-diméthyl-n-propylamine, la (+/-)1-benzyloxyméthyl-1-phényl-N,N-diméthyl-n-propylamine la (+/-) 1-[(3,4,5-triméthoxy)benzyloxyméthyl]-1-phényl-N-méthyl-n-propylamine et la (+/-) 1-p. chlorobenzyloxyméthyl-1-p. methoxyphényl-N,N-diméthyl-n-éthylamine qui sont respectivement les produits des exemples 1, 3, 12 et 13 décrits et annoncés préférés à ce brevet.

En outre, et également au titre de produit de comparaison il a été utilisé :

- l'antipode lévogyre du composé de l'invention la (-) 1-[(3,4,5 -triméthoxy) benzyloxméthyl]-1-phényl-N,N-diméthyl-N- propylamine dont la préparation est décrite en partie expérimentale du texte,
- la Trimébutine (DCI) qui est le (+/-) 3, 4, 5-triméthoxy benzoate de diméthylamino-2-phényl-2-n butanol, objet du brevet français n° 2 369 M et qui est de structure chimique voisine de celle du racémique correspondant au produit de l'invention
- le composé codé U. 50,488 précédemment cité actif sur la vidange gastrique et qui est le trans-3,4-dichloro-N-méthyl-N- [2-(1-pyrrolidinyl) cyclohexy]phénylacétamide annoncé analgésique d'action centrale et antiépileptique (Pharmaprojects - mise à jour Août 1988).

Enfin, dans ces essais la morphine a été utilisée comme substance analgésique de référence.

Ainsi l'activité de l'énantiomère est comparée à celle des composés racémiques. Toutefois, il est connu que dans les essais pratiqués, les récepteurs mis en jeu sont stéréo-sélectifs et qu'ils ne sont sensibles qu'à l'activité d'un seul énantiomère, l'autre étant inactif, comme il est décrit dans "CRC Handbook of stéréoisomèrs" Ed. Donald F. Smith. 1984, p. 401 à 440 : "Opiates agonists and antagonists : "Pharmacological, behavorial, and neurochemical effects of stereoisomers". Dans les essais réalisés et à propos des comparaisons, il est tenu compte de ce fait et pour un composé racémique l'expression du résultat de l'essai sera multiplié par deux

ou encore pour un résultat de valeur donnée la dose de produit racémique responsable de l'effet sera divisée par deux avant comparaison avec le résultat du composé de l'invention.

Tel qu'il a été reporté précédemment l'étude de l'affinité de liaison des produits aux récepteurs opiacés mu, delta et kappa a été réalisé selon la technique décrite par F. Roman et coll. (déjà cité).

Les résultats sont présentés au tableau 1 qui suit et sont exprimés en $CI_{50}$ qui sont les concentrations nanomolaires des produits en solution capables d'inhiber 50 % des liaisons d'un ligand radioactif spécifique lié au récepteur considéré.

**Tableau 1 :**

<u>Affinité de liaison du composé et des produits de comparaison aux récepteurs mu, delta et kappa.</u>

| Composé à l'essai | Rec. mu | Rec.delta | Rec.kappa |
|---|---|---|---|
| - <u>Produit de l'invention</u> : | | | |
| Enantiomère + | 196 | 540 | 232 |
| - Antipode - Enantiomère - | 580 | 12 320 | 2 630 |
| - Ex. 1 de US n° 4,301,163 | 1 110 | 7 800 | 1 110 |
| - Ex. 3 " " | 190 | 14 700 | 560 |
| - Ex.12 " " | 360 | 2 250 | 2 280 |
| - Ex.13 " " | 250 | 4 390 | 900 |
| - Trimébutine | 146 | 1 750 | 1 460 |
| - U. 50,488 | 1 028 | 14 610 | 66 |
| - Morphine | 7 | 152 | 127 |

En considérant ces résultats et même en pratiquant pour les composés racémiques la correction précédemment proposée et qui n'est pas rapportée au tableau 1, il est remarquable que l'énantiomère dextrogyre de l'invention soit le seul composé avec la morphine à montrer une affinité sur les trois récepteurs étudiés et ce avec une intensité non négligeable.

Par contre, en considérant le seul récepteur mu, les composés des exemples 3, 12 et 13 du brevet des Etats-Unis d'Amérique de même que la Trimébutine sont autant sinon plus actifs. Il en est de même pour le composé U. 50,488 si l'on considère le seul récepteur kappa.

L'affinité de composés aux récepteurs opiacés "in vitro" étant souvent représentatifs de propriétés pharmacologiques analgésiques (CRC Handbook of Stereoisomers - déjà cité) les produits sont engagés dans un test "in vivo" approprié à mettre en évidence cette activité analgésique par voie générale.

Ce test réalisé chez la souris selon une technique dérivée de celle de Koster R. (Fed. Proc. 1959, 18, p. 412: consiste à étudier l'influence des produits administrés par voie orale à l'animal sur les manifestations douloureuses provoquées par l'administration par voie intrapéritonéale d'une solution d'acide acétique.

Pour cela, les animaux par lots de 10, sont mis à jeun de nourriture et de boisson 20 heures avant le test. Ils reçoivent alors en solution aqueuse les produits à étudier à raison de 2 ml de solution par 100 g de poids corporel, puis 10 minutes après, une injection intrapéritonéale de 0,25 ml d'une solution d'acide acétique à 0,5 % (v,v) maintenue à 37°C. Trois minutes après cette injection, le nombre de crampes abdominales présentées par les animaux commence à être compté et ce durant 10 minutes. Les animaux sont considérés comme analgésiés lorsqu'ils présentent un nombre de crampes inférieur à la moitié de la moyenne des crampes présentées par les animaux du lot témoin. Dans le test les produits ont été administrés à la dose de 50 mg par kg, la morphine utilisée comme référence provoque administrée par voie orale à raison de 10 mg par kg, un

effet analgésique de 78 %.

Le tableau 2 reporte les résultats de cette étude. Ils mettent en évidence et confirment la particularité du produit de l'invention qui se montre très peu analgésique par rapport aux autres composés étudiés. Cette constatation est d'autant plus marquée si pour ces composés racémiques il est appliqué la correction proposée. Dans ce cas, l'énantiomère dextrogyre de l'invention (17,60 %) est pratiquement 3 fois moins analgésique que la Trimébutine (24,3 x 2 = 48,6 %) et 5 fois moins (44,0 x 2 = 88,0 % que le composé de l'exemple 12 du brevet des Etats-Unis d'Amérique.

Tableau 2 :

Activité analgésique "in vivo"
(50 mg/kg - voie orale)

| Composé à l'essai | % analgésie (p) |
|---|---|
| - Produit de l'invention - | |
| Enantiomère + | 17,6 (*) |
| - Ex. 3 de US n° 4,301,163 | 27,6 (*) |
| - Ex. 12 " " | 44,0 (***) |
| - Ex. 13 " " | 27,9 (***) |
| - Trimébutine | 24,3 (**) |
| - U. 50,488 | 90,0 (***) |

(p) Probabilité - Test T de Student
(*) $p < 0,05$
(**) $p < 0,01$
(***) $p < 0,001$

La recherche de l'activité locale du produit de l'invention sur les récepteurs opiacés de la muqueuse gastrique a été effectuée selon une méthode inspirée de celle décrite par M. Gué et Coll. (déjà cité).

Les auteurs étudient chez le chien, l'action de composés opiacés de type mu et de type kappa après administration par voie orale. Aux faibles doses utilisées ils n'observent pas d'effet pour les composés mu agoniste alors que les composés de type kappa agoniste (dont le U. 50,488) favorisent l'évacuation gastrique de la phase solide du repas chez le chien, cet effet étant par ailleurs inhibé par des composés opiacés antagonistes : la naloxone et le MR 2266 qui sont connus être respectivement des antagonistes des récepteurs mu et des récepteurs kappa (D. Römer et coll., Life Sciences, 31, p. 1217-1220, 1982).

D'après cette étude, les auteurs avancent que les composés opiacés de type kappa agoniste comme le Tifluadom et le U. 50,488, lorsqu'ils sont administrés par voie orale modifient l'évacuation gastrique par leur action locale.

Dans l'hypothèse de montrer une action de ce type du produit de l'invention celui-ci a été engagé dans cet essai avec pour produits de comparaison, son antipode lévogrye et la Trimébutine qui est connue pour son activité régulatrice du fonctionnement gastro-intestinal.

Le principe de l'essai consiste à mesurer le contenu gastrique de l'animal, une heure après l'ingestion d'un repas comprenant 400 g de matières solides à 21,7 % de matières sèches composées par :

- protéines 7,7 %,
- lipides 4,5 %,
- hydrates de carbone 6,9 %,
- sels minéraux 2,6 %,

et 20 g de foie de mouton contenant de la cyanocobalamine radiomarquée. Le repas est consommé en 5 minutes environ par les animaux. Après une heure, le contenu gastrique est recueilli par l'intermédiaire d'une canule mise en place à 10 cm environ du pylore avant l'expérience.

L'échantillon recueilli est pesé et homogénéisé. Sa radioactivité est mesurée et l'expression de la vidange gastrique exprimée en pourcentage est calculée selon la formule :

$$\% \text{ vidange} = \frac{(\text{cpm1 . P1} - \text{cpm2 . p}) \times 100}{\text{cpm1 . P1}}$$

dans laquelle :

cpm1 est la radioactivité par gramme de foie marqué mélangé,
P1 est le poids de foie marqué mélangé au repas,
cpm2 est la radioactivité par gramme d'échantillon recueilli après une heure,
p est le poids de cet échantillon recueilli après une heure.

Pratiquement, chaque essai est réalisé avec trois chiens et consiste à leur administrer 20 minutes avant l'ingestion du repas précédemment décrit, une gélule contenant le produit à l'essai à une dose de 0,25 mg par kg d'animal ou bien une gélule placebo qui sert à déterminer le phénomène témoin de la vidange sur l'animal. Les trois chiens recoivent chaque traitement deux fois dans un ordre randomisé. Enfin, dans une dernière série d'essais, 3 chiens reçoivent une administration par voie intraveineuse d'une solution de MR 2266 ou de naloxone à raison de 0,1 mg par kg de poids. Le repas est présenté et ingéré 20 minutes après l'administration de la gélule contenant le produit à l'essai. Les valeurs d'évacuation des phases solides sont mesurées comme il est décrit plus haut et comparées par le test de Mann et Whitney (U test). La différence par rapport aux valeurs témoins est considérée significative lorsque p et < ou = à 0,05.

L'effet des produits à l'essais est objectivé par le % de variation qui est calculé selon la relation :

$$\% \text{ variation} = \frac{(\% \text{ évac. essai} - \% \text{ évac. témoin}) \times 100}{\% \text{ évac. témoin}}$$

Les résultats obtenus pour cette étude sont rapportés au tableau 3.

Tableau 3 :

Effet des produits sur l'évacuation gastrique des solides chez le chien

| Produit à l'essai (*) | Variation en % |
| --- | --- |
| - Produit de l'invention - | |
| Enantiomère (+) | + 110 % |
| - id. + naloxone | + 5 % |
| - id + MR 2266 | + 14 % |
| - Antipode - Enantiomère (-) | - 26 % |
| - Trimébutine | - 4 % |

* A l'exception de la Trimébutine qui a été administrée à une dose de 5 mg/kg les produits ont été, conformément au protocole précédemment décrit, administrés à raison de 0,25 mg/kg.

L'action du produit de l'invention est indéniable : une heure après son administration son effet double l'évacuation gastrique des solides chez le chien alors que l'effet de son antipode levogyre est quatre fois moins important. Par ailleurs, l'action observée est inhibée à la fois par la naloxone qui est plus particulièrement antagoniste des récepteurs mu et le MR 2266 antagoniste des récepteurs kappa ce qui permet d'attribuer l'effet de vidange à une activité générale de type agoniste sur les récepteurs opiacés locaux de la muqueuse gastrique et plus particulièrement sur les récepteurs mu et kappa.

L'activité antispasmodique du produit de l'invention sous forme de son D (-) tartrate a été recherchée "in vitro" sur le duodénum de rat qui est une partie essentielle du tractus intestinal pour cet animal.

Sur un fragment de ce tissu, l'étude consiste à provoquer des contractions par un agent spasmogène qui dans cette étude est le chlorure de baryum, puis à déterminer la concentration de solution ($CI_{50}$) du produit capable d'inhiber 50 % de l'amplitude des contractions provoquées.

Les résultats de l'étude sont présentés au Tableau 4

**Activité antispasmodique "in vitro" sur le duodénum de rat**

(Spasmogène $BaCl_2$)

| Produit à l'essai | $CI_{50}$ mol. litre |
|---|---|
| - Produit de l'invention - | |
| Enantiomère (+) | 1,70. $10^{-5}$ |
| - Papavérine | 2,74. $10^{-5}$ |
| - Morphine | Inactive à $10^{-4}$ |

Alors que la morphine est inactive dans cet essai le produit de l'invention montre une activité légèrement supérieure à celle de la papavérine qui est considérée comme composé de référence d'activité spasmolytique.

Enfin, l'étude de la toxicité aigüe du produit de l'invention montre également un intérêt notamment vis à vis de son antipode lévogyre.

Cette toxicité a été étudiée par voie orale chez la souris mâle. Les produits à l'essai ont été administrés à raison de 2 ml de leur solution aqueuse pour 100 g en poids d'animaux. Les animaux sont ensuite observés durant les trois heures qui suivent l'administration puis quotidiennement durant quatorze jours, moment auquel ils sont sacrifiés puis autopsiés.

Les $DL_{50}$ (doses léthales qui provoquent la mort de 50 % des animaux) ont été calculées selon la méthode de Reed J.L. et Muench H. (Am. J. Hyg. 1939, 27, p. 493). Les résultats obtenus figurent au tableau 5 qui suit et montrent que le produit de l'invention est environ deux fois moins toxique dans cet essai que son antipode lévogyre.

**Tableau 5 :**

**Toxicité aigüe par voie orale chez la souris**

| Produit à l'essai | $DL_{50}$ mg/kg |
|---|---|
| - Produit de l'invention - | |
| Enantiomère (+) | 407 |
| - Antipode - Enantiomère (-) | 255 |

Les études qui viennent d'être décrites montrent sans équivoque l'intérêt du produit de l'invention dont les propriétés particulières qui consistent, d'une part, en une activité locale de type agoniste sur les récepteurs opiacés de la muqueuse gastrique et, d'autre part, en une activité antispasmodique notamment au niveau intestinal justifient à double titre son utilité à traiter les dysfonctionnements digestifs auxquels la motricité gastrique est liée, comme le reflux gastro oesophagien, les ulcères, la dyspepsie, les gastrites, la colopathie fonctionnelle et les dyskinésies biliaires.

A titre de principe actif du médicament selon l'invention, il peut être utilisé l'amine libre qui est la (+)-1[(3,4,5 triméthoxy) benzyloxyméthyl]-1-phényl-N,N-diméthyl -n-propylamine ou ses sels d'addition avec les acides minéraux ou organiques pharmaceutiquement acceptables. A ce titre les acides chlorhydrique, bromhydrique,

phosphorique, sulfurique ainsi que les acides acétique, citrique, gluconique, maléique sont utilisés. En ce qui concerne les acides organiques l'acide D (-) tartrique permet d'obtenir le sel d'addition préféré avec le composé de l'invention car, d'une part, sa purification par cristallisation dans l'éthanol est aisée et efficace et, d'autre part, il est hydrosoluble, et donc approprié à la préparation de formes pharmaceutiques aqueuses, sans être hygroscopique. Il présente la stabilité physicochimique nécessaire à son utilisation comme principe actif dans l'industrie pharmaceutique.

L'invention vise aussi un procédé de préparation de l'amino éther oxyde dextrogyre et de son D (-) tartrate qui peuvent être obtenus :

- soit par le procédé préféré apparenté à celui présenté au brevet des Etats-Unis d'Amérique n° 4,301,163 et qui consiste à faire réagir le (+) 2-diméthylamino-2-phényl-n-butanol préparé selon la méthode décrite à la demande de brevet japonais publiée sous le n° 16416/1980 le 1er Mai 1980, avec un 3, 4, 5 triméthoxybenzyl halogénure de formule

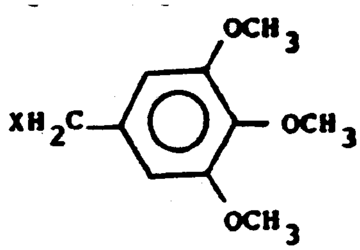

dans lequel X est un atome d'halogène qui est le chlore, le brome ou l'iode.

Plus précisément, on prépare dans un premier temps l'alcoolate métallique de l'amino alcool dextrogyre par réaction avec un métal alcalin comme le sodium, le potassium ou leurs hydrures ou leurs amidures. Cette réaction est couramment réalisée dans des solvants aromatiques comme le benzène, le toluène, les xylènes ou dans des solvants éthérés comme l'éther diéthylique, le dioxanne, le tétrahydrofuranne ou encore dans des solvants comme le dimethylsulfoxyde, le diméthylformamide, l'acétonitrile, le diméthylacétamide, l'hexaméthylphosphorotramide. La réaction de salification est effectuée en utilisant pour une mole d'alcool de 0,7 à 1,5 moles d'agent métallique et, de préférence de 0,9 à 1,2 moles de cet agent. La température de salification est comprise entre 0 et 140°C et plus particulièrement entre 20 et 110°C.

Le sel métallique de l'amino alcool est formé après 30 minutes à 6 heures de réaction puis il est condensé avec l'halogénure de 3, 4, 5 triméthoxybenzyle à une température comprise entre 0 et 140°C et plus favorablement 70 à 110°C. La durée de condensation est de 1 à 24 heures et plus favorablement entre 2 et 6 heures, l'état d'avancement de la réaction pouvant être suivi par chromatographie sur couches minces. La séparation du produit formé étant réalisée par des méthodes habituelles décrites dans l'exemple illustratif.

L'amino éther oxyde dextrogyre obtenu peut être salifié avec les acides appropriés déjà cités. D'une façon préférée cette opération est effectuée par l'acide D (-) tartrique que l'on utilise à raison de 0,75 à 1,25 mole par mole de produit à salifier. La réaction est réalisée dans des solvants comme des cétones ou des alcools de faible poids moléculaire. L'éthanol est préféré. Il est utilisé à raison de 3 à 10 volumes par partie en poids d'isomère. Ainsi pour une mole, on ajoute habituellement de 5 à 7 volumes par poids d'éthanol et de 0,95 à 1,05 mole d'acide D (-) tartrique. La réaction est conduite à une température comprise entre 20 et 80°C durant 15 minutes à 5 heures et plus précisément entre 30 minutes et une heure à une température comprise entre 35 et 55°C. La cristallisation du sel est complète après 48 heures à 20°C et permet d'obtenir par filtration le D (-) tartrate de l'isomère dextrogyre de l'invention dans un état de pureté satisfaisant à son utilisation thérapeutique. Malgré tout, s'il est nécessaire, le produit est purifié par recristallisation dans l'éthanol.

- soit par résolution de l'amino éther oxyde racémique préparé comme à l'exemple 2 du brevet des Etats Unis d'Amérique n° 4,301,163.

D'une façon conventionnelle cette résolution est effectuée par séparation des diastéréoisomères formés après salification avec des acides optiquement actifs. Ce procédé est couramment pratiqué. On trouve une compilation particulièrement complète des réactifs et de leur emploi dans "Optical résolution Procedures for Chemical compounds" - Vol. 1 : Amines and related compounds. Paul Newman - 1978.

Plus précisément, le procédé consiste à salifier en solution dans un solvant qui peut être une cétone ou un alcool de point d'ébullition inférieur ou voisin de 80°C, la propylamine racémique correspondante par un acide organique dextrogyre (+) en un sel d'acide (+) propylamine (-) insoluble ; à écarter ce sel insoluble par filtration pour obtenir une solution contenant à la fois de l'acide (+) et l'énantiomère (+) de la n-propylamine, à évaporer le solvent de cette solution jusqu'à siccité pour obtenir un résidu, à dissoudre le résidu dans l'eau et alcaliniser la solution jusqu'à un pH voisin ou supérieur à 10 par addition de solution d'hydroxyde alcalin ou

alcalino terreux ou encore d'ammoniaque, à extraire la n-propylamine dextrogyre libérée par un solvant organique puis évaporer ce solvant organique d'extraction pour obtenir la (+) n-propylamine cherchée.

Pour la mise en oeuvre de cette méthode de résolution, les énantiomères de l'acide tartrique sont préférés. Leur emploi consiste dans une première étape à former les diastéréoisomères avec l'acide L (+) tartrique, à éliminer par filtration le diastéréoisomère formé entre l'acide et l'énantiomère lévogyre qui cristallise, puis à traiter le filtrat de façon à libérer de son sel et à isoler l'isomère dextrogyre de la n-propylamine qui est l'objet de l'invention et, finalement, à préparer son sel d'addition avec l'acide D (-) tartrique, sel qui éventuellement peut être purifié comme il est décrit précédemment.

Ainsi pour effectuer la résolution d'une mole de composé racémique on utilise de 0,6 à 1,5 moles d'acide L (+) tartrique qui sont mis à réagir pour salification de l'amine dans un solvant qui de préférence est un alcool ou une cétone de faible poids moléculaire et de point d'ébullition voisin ou inférieur à 80°C. Il est préféré que ce solvant soit anhydre. L'éthanol absolu est le plus fréquemment utilisé à raison de 3 à 30 parties en volume par partie en poids de composé à traiter.

La salification est réalisée en chauffant la solution obtenue de 5 minutes à 1 heure au reflux du solvant puis en laissant cristalliser par refroidissement le diastéréoisomère composé d'acide L (+) tartrique et de l'antipode lévogyre du composé de l'invention.

Ce sel qui est un sous produit est éliminé par filtration. Le filtrat est ensuite traité de façon appropriée pour obtenir l'énantiomère dextrogyre de l'invention. A cet effet, on élimine le solvant par distillation, reprend le résidu par une solution alcaline en quantité suffisante pour obtenir un pH voisin ou supérieur à 10 puis extrait le produit de l'invention dans un solvant approprié tel que l'éther ou le chlorure de méthylène.

Après évaporation de ce solvant d'extraction, l'énantiomère dextrogyre de l'invention est purifié comme il a été précédemment décrit, par formation d'un sel d'addition notamment avec l'acide D (-) tartrique qui est préféré puis purification par cristallisation de ce sel.

Ces procédés sont illustrés de façon non limitative en partie expérimentale par des exemples qui décrivent leur mise en oeuvre et les caractéristiques des produits de l'invention obtenus.

L'utilité du produit de l'invention et de ses sels a été montrée pour traiter divers dysfonctionnements de la motricité digestive. Selon la nature et la gravité de l'affection à traiter la dose thérapeutique quotidienne est comprise entre 5 et 1000 mg et plus favorablement entre 25 et 500 mg de produit pouvant être absorbé en une ou plusieurs prises.

Le produit est présenté sous des formes médicamenteuses conventionnelles telles que des comprimés, des gélules, des suppositoires, des solutés ou des suspensions buvables ou pulvérisables ou encore injectables.

Pour les formes galéniques dites "sèches" la quantité de principe actif peut intervenir à raison de 5 à 80 % en poids du produit terminé, l'ensemble des excipients intervenant pour leur part à raison de 95 à 20 % de ce poids. Dans les formes dites "aqueuses" (suspensions et solutés) le principe actif peut intervenir à raison de 0,1 à 20 % en poids de la composition, l'eau et les divers adjuvants représentant de 99,9 à 80 % du pois total de la préparation terminée.

A titre d'illustration, les préparations de solutés injectables à 0,5 % (p/v) et celle de comprimés enrobés dosés à 100 mg par unité de (-) tartrate du composé de l'invention sont présentés

Formulations.

* Solutés injectables à 0,5 % (p/v)

Composés pour la préparation de 100 ml de soluté :

| | |
|---|---|
| - (-) tartrate de (+) 1-[(3,4,5-triméthoxy) benzyloxyméthyl]-1-phényl-N,N-diméthyl-n-propylamine | 0,500 g |
| - chlorure de sodium officinal | 0,850 g |
| - eau distillée pour préparations injectables q.s.p | 100,0 ml |

Préparation :

Les composés sont solubilisés dans environ 95 % de la quantité d'eau distillée prévue pour la préparation,

à une température voisine de 20°C et sous agitation. La solution obtenue est filtrée sur membrane de porosité 22 microns puis le filtrat complété au volume exact avec de l'eau distillé également filtrée. La solution est conditionnée à raison de 5 ml par ampoule, celles ci sont ensuite scellées puis stérilisées à 121°C durant 30 minutes.

* Comprimés enrobés à 100 mg de principe actif par unité.

Formule unitaire :

| | |
|---|---|
| - (-) tartrate de (+) 1-[(3,4,5-triméthoxy) benzyloxyméthyl]-1-phényl-N,N-diméthyl-n-propylamine | 100,0 mg |
| - phosphate dicalcique | 30,0 mg |
| - lactose | 132,0 mg |
| - amidon de maïs | 80,0 mg |
| - carboxyméthylamidon | 10,0 mg |
| - polyvinylpyrrolidone 25 | 16,0 mg |
| - cellulose microcristalline | 20,0 mg |
| - stéarate de magnésium | 4,0 mg |
| - talc | 8,0 mg |
| - hydroxypropyl méthyl cellulose | 4,4 mg |
| - bioxyde de titane | 1,1 mg |
| pour un total de | 405,5 mg |

Préparation :

Dans un mélangeur-malaxeur on introduit :

| | |
|---|---|
| - principe actif | 1750 g |
| - phosphate dicalcique | 525 g |
| - lactose | 2320 g |
| - amidon de maïs | 1400 g |
| - carboxyméthylamidon | 175 g |

on ajoute au mélange obtenu, pour mouillage, une solution de 105 g de polyvinylpyrrolidone 25 dans 770 ml d'eau purifiée. Le mélange est granulé sur un appareil équipé d'une grille d'ouverture de 2 mm, les grains sont séchés en étuve à 50°C puis calibrés par passage sur une grille d'ouverture de 1 mm.
Dans un mélangeur, à 6365 g de ces grains on ajoute 346 g de cellulose microcristalline, 69 g de stéarate de magnésium et 139 g de talc. Après mélange le produit est comprimé à raison de 401,5 mg par unité puis les comprimés obtenus enrobés en turbine à 40°C par une suspension aqueuse de bioxyde de titane et d'hydroxypropyl méthyl cellulose pour obtenir finalement des comprimés enrobés terminés à un poids moyen de 406,7 mg de poids unitaire et contenant chacun 100,0 mg du (-) tartrate du produit de l'invention.

PARTIE EXPERIMENTALE

Procédés de préparation.

* Exemple 1 (préféré)

a) (+) 1-[(3,4,5-triméthoxy) benzyloxyméthyl]-1-phényl- N,N-diméthyl-n-propylamine,

Dans un réacteur protégé de l'humidité et sous atmosphère d'azote, on introduit 60 ml de dioxanne anhydre puis 6,20 g d'hydrure de sodium en suspension à 80 % (p/p) dans la paraffine (0,207 mol).

Sous agitation et sans dépasser 50°C, il est introduit 40,0 g (0,207 mol) de (+) 2-diméthylamino-2-phényl

butanol $(\alpha)_D$ = + 7,9° ; C = 1, éthanol). La suspension est maintenue sous agitation à 40°C durant 45 minutes puis refroidie à une température voisine de 20°C.

En deux heures, sans dépasser 50°C, on introduit 44,9 g (0,207 mol) de 3,4,5-triméthoxybenzyl chlorure avec 5 ml de dioxanne anhydre. Le milieu réactionnel est chauffé à 70-75°C et maintenu 4 heures à cette température. Après refroidissement, le mélange est abandonné une nuit puis on ajoute lentement 200 ml d'eau en maintenant à une température inférieure à 20°C. Après acidification par l'acide sulfurique jusqu'à pH1, on extrait avec 60 ml de toluène.

La phase toluénique est séparée et écartée. La phase acide est alcalinisée par une solution concentrée de lessive de soude puis extraite par 2 fois 150 ml de chlorure de méthylène.

Les phases organiques réunies sont lavées à l'eau puis déshydratées sur $Na_2SO_4$. Le solvant est distillé. Le produit est obtenu sous forme d'une huile visqueuse jaune pâle.

Poids : 70,30 g    Rdt : 91 %

1H - RMN - $CDCl_3$ (60 MHz, TMS int) - Déplacements chimiques en p.p.m. : 0,70 (triplet, 3H) ; 1,95 (quadruplet, 2H) ; 2,32 (singulet, 6H) 3,90 (singulet, 11H) ; 4,55 (singulet, 2H) ; 6,65 (singulet, 2H) 7,35 (massif, 5H)

$[\alpha]_D$ = + 16,5° (c = 6, éthanol)

b) Salification par l'acide D (-) tartrique,

Dans un réacteur, on introduit 3,50 g (9,38 mmol) de l'énantiomère dextrogyre précédent, 1,37 g (9,13 mmol) d'acide D (-) tartrique et 21,0 ml d'éthanol absolu.

Le mélange est chauffé sous agitation à 50°C et maintenu 30 minutes à cette température. La solution est progressivement refroidie en 16 heures jusqu'à 10°C environ. Les cristaux sont filtrés et séchés sous vide à 50°C jusqu'à poids constant.

Poids : 4,06 g    Rdt = 85 %

F = 147°C    $[\alpha]_D^{25}$ = + 14,5°    (c=5, HClN)

Un prélèvement de ce produit est recristallisé dans l'éthanol bouillant (5 volumes par partie en poids). Les cristaux obtenus ont un point de fusion et un pouvoir rotatoire identique au produit avant traitement. En conséquence, les valeurs du point de fusion et du pouvoir rotatoire mentionnés ci-dessus sont considéré comme caractéristiques du produit purifié.

∗ Exemple 2 -

a) Résolution du composé racémique

Dans un réacteur protégé de l'humidité, on introduit 210 ml d'éthanol absolu, 30,0 g (80,3 mmol) de (+/-) 1-[(3,4,5- triméthoxy) benzyloxyméthyl]-1-phényl-N,N-diméthyl-n-propylamine. On ajoute ensuite 9,6 g (64,2 mmol) d'acide L (+) tartrique puis on chauffe le mélange sous agitation jusqu'au reflux que l'on maintient 15 minutes.

La solution obtenue est progressivement refroidie sous agitation jusqu'à 20°C puis abandonnée une nuit.

L'insoluble est filtré et écarté. Il contient essentiellement le L (+) tartrate de l'énantiomère levogyre antipode du produit de l'invention, qui est purifié pour échantillon de comparaison par recristallisation dans l'éthanol absolu à raison de 5 volumes par partie en poids de produit, jusqu'à pouvoir rotatoire constant :

$[\alpha]_D$ = - 14,5° (c = 5, HCl N)

Le filtrat alcoolique de la première cristallisation qui contient le produit de l'invention est évaporé sous vide et le résidu dissout dans 160 ml d'eau.

La solution est alcalinisée jusqu'à pH 10 avec une solution concentré d'hydroxyde de sodium puis extraite par deux fois 120 ml de chlorure de méthylène. Les phases organiques réunies sont lavées à l'eau puis séchées sur $Na_2SO_4$. Après élimination du chlorure de méthylène par distillation, on obtient un résidu huileux jaune pale enrichi en isomère dextrogyre.

Poids : 16,50 g    Rdt pondéral : 55 %

$[\alpha]$ = + 14,7°C (c = 6, éthanol) - pureté optique = 89 %

b) Salification par l'acide D (-) tartrique,

On procède selon le mode opératoire décrit en b) de l'exemple 1 et à partir de 12,6 g (33,7 mmol) du produit précédent et de 5,05 g (33,7 mmol) d'acide D (-) tartrique dans 110 ml d'éthanol anhydre. Le produit cristallisé est filtré et séché.

Poids : 13,8 g Rdt = 78 %
F = 143-145°C $[\alpha]_D^{25}$ = + 13,8° (c = 5, HClN)
pureté optique = 95 %

Le produit est recristallisé dans 65 ml d'éthanol au reflux. Après lent refroidissement à 20°C sous agitation, les cristaux sont filtrés et séchés sous vide jusqu'à poids constant

Poids : 12,4 g Rdt = 90 %
F = 147°C $[\alpha]_D^{25}$ = + 14,5° (c=5, HClN)

Un échantillon du produit est traité en milieu alcalin et extrait au chlorure de méthylène. Le résidu huileux jaune pale obtenu après évaporation présente un pouvoir rotatoire $(\alpha)_D$ de + 16,6° à une concentration de 6 % (p/v) dans l'éthanol. Cette valeur est considérée caractéristique, dans les conditions de la détermination, de la pureté optique de l'isomère dextrogyre objet de l'invention.

## Revendications

### Revendications pour les Etats contractants suivants : AT, BE, CH, DE, FR, GB, IT, LI, LU, NL, SE

1. La (+) 1 [(3,4,5 triméthoxy) benzyloxyméthyl]-1-phényl-N,N-diméthyl-n-propylamine de formule

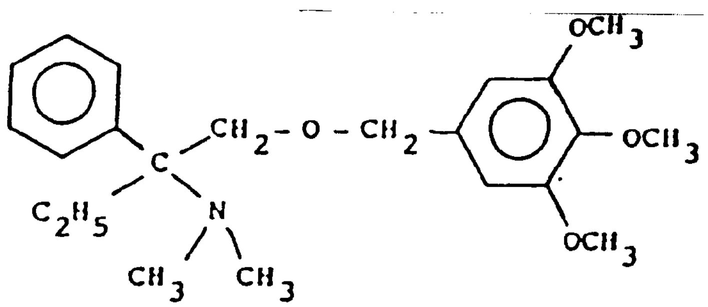

et ses sels d'addition avec des acides thérapeutiquement acceptables.

2. Le D (-) tartrate de (+) 1-[(3,4,5 triméthoxy) benzyloxyméthyl]-1-phényl-N,N-diméthyl-n-propylamine

3. Procédé de préparation de la (+) 1-[(3,4,5 triméthoxy) benzyloxyméthyl]-1-phényl-N,N-diméthyl-n-propylamine caractérisé en ce qu'il consiste à faire réagir le (+) 2-diméthylamino -2-phényl-n-butanol de formule

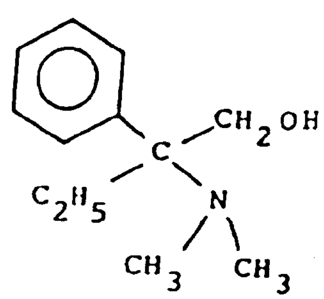

sur un halogènure de 3,4,5-triméthoxybenzyle de formule

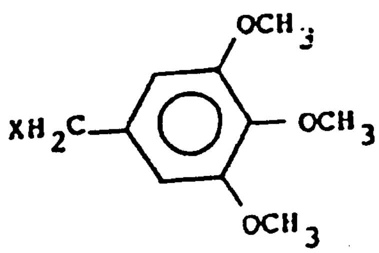

dans laquelle X est un halogène, de préférence le chlore, le brome ou l'iode.

4. Procédé de préparation de la (+) 1-[(3,4,5 triméthoxy) benzyloxyméthyl]-1-phényl-N,N-diméthyl-n-propylamine caractérisé en ce qu'il consiste à salifier en solution dans un solvant la propylamine racémique correspondante par un acide organique dextrogyre (+) en un sel d'acide (+) propylamine (-) insoluble ; à écarter ce sel insoluble par filtration pour obtenir une solution contenant à la fois de l'acide (+) et l'énantiomère (+) de la n-propylamine, à évaporer le solvant de cette solution jusqu'à siccité pour obtenir un résidu, à dissoudre le résidu dans l'eau et alcaliniser la solution jusqu'à un pH voisin ou supérieur à 10 par addition de solution d'hydroxyde alcalin ou alcalino terreux ou encore d'ammoniaque, à extraire la n-propylamine dextrogyre libérée par un solvant organique puis évaporer ce solvant organique d'extraction pour obtenir la (+) n- propylamine cherchée

5. Procédé suivant la revendication 4 qui consiste pour salifier la propylamine racémique dans un solvant à utiliser l'acide L (+) tartrique.

6. Procédé suivant la revendication 3 ou la revendication 4, caractérisé en ce qu'il consiste pour préparer un sel de la (+) n-propylamine cherchée à la salifier par un acide pharmaceutiquement acceptable,

7. Médicament utile en gastroentérologie caractérisé en ce qu'il comprend l'amine dextrogyre ou un de ses sels suivant les revendications 1 et 2 ayant une action locale sur les récepteurs opiacés du tractus gastrointestinal, sensiblement dénuée d'actions centrales et ayant également une action spasmolytique.

**Revendications pour l'Etat contractant suivant : ES**

1. Procédé de préparation de la (+) 1-[(3,4,5 triméthoxy) benzyloxyméthyl]-1-phényl-N,N-diméthyl-n-propylamine de formule

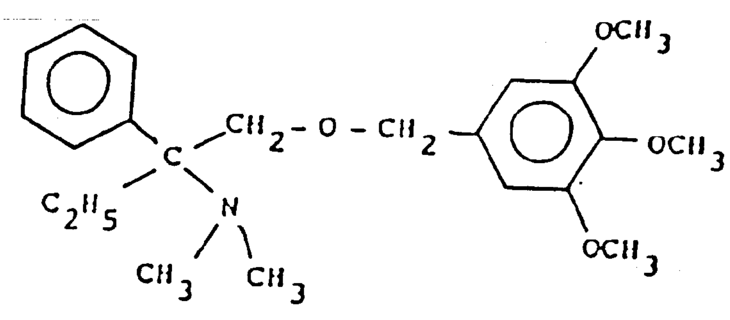

et de ses sels d'addition avec des acides thérapeutiquement acceptables, notamment du D (-) tartrate de (+) 1-[(3,4,5 triméthoxy)benzyloxyméthyl]-1-phényl-N,N-diméthyl-N-propylamine, caractérisé en ce qu'il consiste à faire réagir le (+) 2-diméthylamino -2-phényl-n-butanol de formule

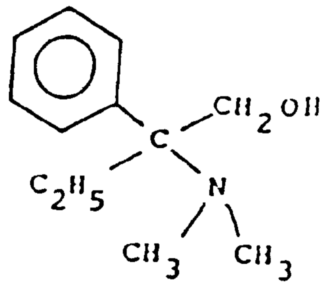

sur un halogènure de 3,4,5-triméthoxybenzyle de formule

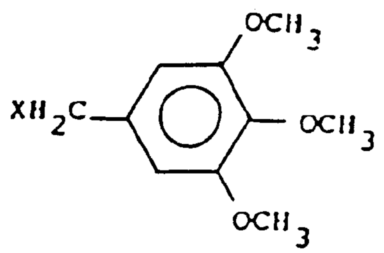

dans laquelle X est un halogène, de préférence le chlore, le brome ou l'iode, et, pour préparer un sel, à salifier par un acide.

2. Procédé de préparation de la (+) 1-[(3,4,5 triméthoxy) benzyloxyméthyl]-1-phényl-N,N-diméthyl-n-propylamine de formule

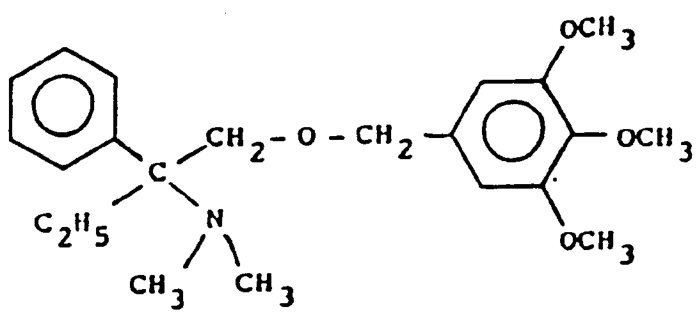

et de ses sels d'addition avec des acides thérapeutiquement acceptables, notamment du D (-) tartrate de (+) 1-[(3,4,5 triméthoxy) benzyloxyméthyl]-1-phényl-N,N-diméthyl-n-propylamine, caractérisé en ce qu'il consiste à salifier en solution dans un solvant la propylamine racémique correspondante par un acide organique dextrogyre (+) en un sel d'acide (+) propylamine (-) insoluble ; à écarter ce sel insoluble par filtration pour obtenir une solution contenant à la fois de l'acide (+) et l'énantiomère (+) de la N-propylamine, à évaporer le solvant de cette solution jusqu'à sicccité pour obtenir un résidu, à dissoudre le résidu dans l'eau et alcaliniser la solution jusqu'à un pH voisin ou supérieur à 10 par addition d'une solution d'hydroxyde alcalin ou alcalino terreux ou encore d'ammoniaque, à extraire la n-propylamine dextrogyre libérée par un solvant organique puis évaporer ce solvant organique d'extraction pour obtenir la (+) n-propylamine cherchée et, pour préparer un sel, à la salifier par un acide.

3. Procédé suivant la revendication 2 qui consiste pour salifier la propylamine racémique dans un solvant à utiliser l'acide L (+) tartrique.

4. Procédé de préparation d'un médicament utile en gastroentérologie, caractérisé en ce qu'il consiste à mélanger l'amine dextrogyre ou un de ses sels préparée suivant les revendications précédentes ayant une action locale sur les récepteurs opiacés du tractus gastrointestinal, sensiblement dénuée d'actions centrales et ayant également une action spasmolytique à un excipient pharmaceutiquement acceptable.

**Revendications pour l'Etat contractant suivant : GR**

1. La (+) 1[(3,4,5 triméthoxy) benzyloxyméthyl]-1-phényl-N,N-diméthyl-n-propylamine de formule

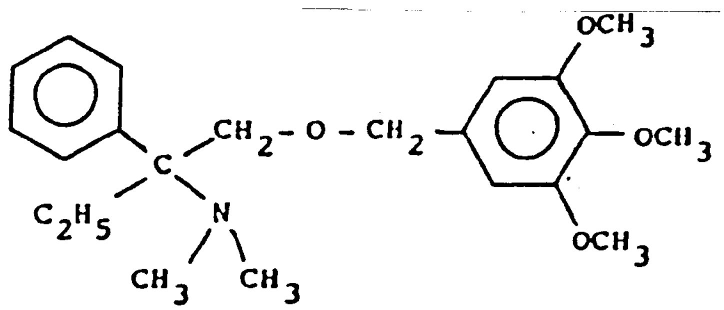

et ses sels d'addition avec des acides.

2. Le D (-) tartrate de (+) 1-[(3,4,5 triméthoxy) benzyloxyméthyl]-1-phényl-N,N-diméthyl-n-propylamine

3. Procédé de préparation de la (+) 1-[(3,4,5 triméthoxy) benzyloxyméthyl]-1-phényl-N,N-diméthyl-n-propylamine caractérisé en ce qu'il consiste à faire réagir le (+) 2-diméthylamino -2-phényl-n-butanol de formule

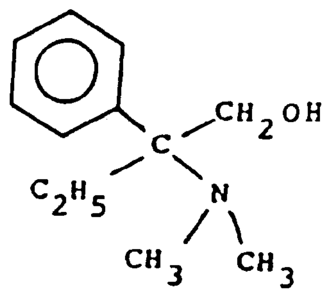

sur un halogènure de 3,4,5-triméthoxybenzyle de formule

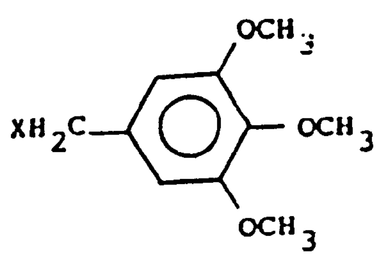

dans laquelle X est un halogène, de préférence le chlore, le brome ou l'iode.

4. Procédé de préparation de la (+) (+) 1-[(3,4,5 triméthoxy) benzyloxyméthyl]-1-phényl-N,N-diméthyl-n-propylamine caractérisé en ce qu'il consiste à salifier en solution dans un solvant la propylamine racémique correspondante par un acide organique dextrogyre (+) en un sel d'acide (+) propylamine (-) insoluble ; à écarter ce sel insoluble par filtration pour obtenir une solution contenant à la fois de l'acide (+) et l'énantiomère (+) de la n-propylamine, à évaporer le solvant de cette solution jusqu'à siccité pour obtenir un résidu, à dissoudre le résidu dans l'eau et alcaliniser la solution jusqu'à un pH voisin ou supérieur à 10 par addition de solution d'hydroxyde alcalin ou alcalino terreux ou encore d'ammoniaque, à extraire la n-propylamine dextrogyre libérée par un solvant organique puis évaporer ce solvant organique d'extraction jour obtenir la (+) n- propylamine cherchée

5. Procédé suivant la revendication 4 qui consiste pour salifier la propylamine racémique dans un solvant à utiliser l'acide L (+) tartrique.

6. Procédé suivant la revendication 3 ou la revendication 4, caractérisé en ce qu'il consiste,pour préparer un sel de la (+) n-propylamine cherchée,à la salifier par un acide pharmaceutiquement acceptable.

7. Procédé de préparation d'un médicament utile en gastroentérologie, caractérisé en ce qu'il consiste à mélanger l'amine dextrogyre ou un de ses sels,préparée suivant les revendications 1 et 2, ayant une action locale sur les récepteurs opiaces du tractus gastrointestinal, sensiblement dénuée d'action centrale et ayant également une action spasmolytique,à un excipient pharmaceutiquement acceptable.

8. L'utilisation de la propylamine telle que définie à la revendication 1 ou de l'un de ses sels acceptables thérapeutiquement pour l'obtention d'un médicament ayant une action locale sur les récepteurs opiaces du tractus gastrointestinal, sensiblement dénuée d'action centrale et ayant également une action spasmolytique.

## Patentansprüche

## Patentansprüche für folgende Vertragsstaaten : AT, BE, CH, DE, FR, GB, IT, LI, LU, NL, SE

1. (+)-1-[(3,4,5-Trimethoxy)-benzyloxymethyl]-1-phenyl-N,N-dimethyl-n-propylamin der nachstehenden Formel

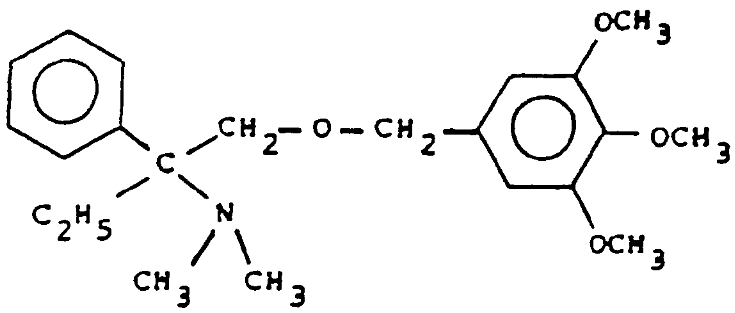

und dessen Additionssalze mit therapeutisch verträglichen Säuren.

2. D-(-)-Tartrat des (+)-1-[(3,4,5-Trimethoxy)-benzyloxymethyl]-1-phenyl-N,N-dimethyl-n-propylamin.

3. Verfahren zur Herstellung von (+)-1-[(3,4,5-Trimethoxy)-benzyloxymethyl]-1-phenyl-N,N-dimethyl-n-propylamin,
dadurch gekennzeichnet, daß
(+)-2-Dimethylamino-2-phenyl-n-butanol der nachstehenden Formel

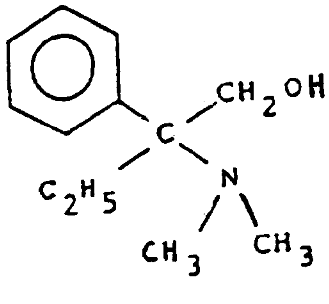

umgesetzt wird mit einem Halogenid des 3,4,5-Trimethoxybenzyl der nachstehenden Formel

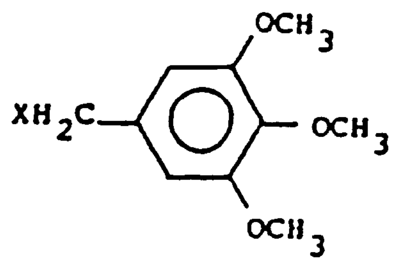

wobei x für Halogen steht, vorzugsweise für Chlor, Brom oder Iod.

**4.** Verfahren zur Herstellung von (+)-1-[(3,4,5-Trimethoxy)-benzyloxymethyl]-1-phenyl-N,N-dimethyl-n-propylamin,
gekennzeichnet durch die Verfahrensschritte:
- aus der Lösung des entsprechenden Propylracemat in einem Lösungsmittel wird durch Zugabe einer rechtsdrehenden organischen Säure "(+)-Säure" das in der Lösung unlösliche Salz "(+)-Säure-(-)-Propylamin" gebildet;
- dieses unlösliche Salz wird durch Filtrieren entfernt, um eine Lösung zu erhalten, die zugleich die (+)-Säure und das (+)-Enantiomere des n-Propylamin enthält;
- diese Lösung wird zur Trockene eingedampft, um das Lösungsmittel zu entfernen und um einen Rückstand zu erhalten;
- der Rückstand wird in Wasser aufgelöst und die erhaltene Lösung wird durch Zugabe einer Alkalihydroxid-, Erdalkalihydroxid oder Ammoniak-Lösung alkalisch gemacht bis zu einem pH-Wert in der Nähe von 10 oder darüber;
- das freigesetzte, rechtsdrehende n-propylamin wird mit einem organischen Lösungsmittel extrahiert, und daraufhin wird dieses organische Extraktionslösungsmittel durch Eindampfen entfernt, um das gesuchte (+)-n-propylamin zu erhalten.

**5.** Verfahren nach Anspruch 4,
dadurch gekennzeichnet, daß
zur Salzbildung mit dem propylamin-Racemat in einem Lösungsmittel L-(+)-Weinsäure zugesetzt wird.

**6.** Verfahren nach Anspruch 3 oder 4,
dadurch gekennzeichnet, daß
zur Salzbildung des gesuchten (+)-n-propylamin dessen Salz mit einer pharmazeutisch verträglichen Säure gebildet wird.

**7.** Arzneimittel, das in der Gastroenterologie brauchbar ist,
dadurch gekennzeichnet, daß
das Arzneimittel als Wirkstoff das rechtsdrehende Amin nach den Ansprüchen 1 und 2 oder eines seiner Salze enthält; und
dieses Arzneimittel eine lokale Wirkung auf die Opiatrezeptoren des Magen-Darm-Traktes - ohne merkliche zentrale Wirkungen - und ferner eine spasmolytische Wirkung aufweist.

**Patentansprüche für folgenden Vertragsstaat : ES**

**1.** Verfahren zur Herstellung von (+)-1-[(3,4,5-Trimethoxy)-benzyloxymethyl]-1-phenyl-N,N-dimethyl-n-propylamin der nachstehenden Formel

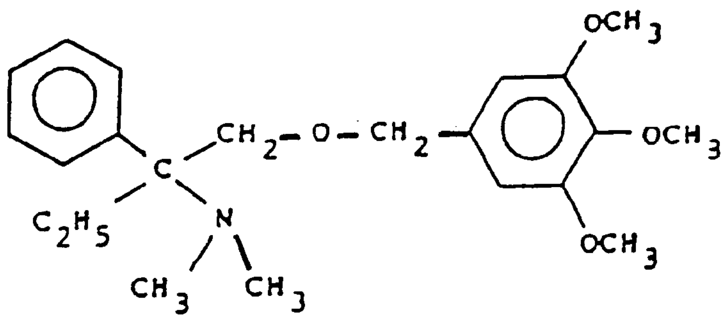

und dessen Additionssalze mit therapeutisch verträglichen Säuren, insbesondere des D-(-)Tartrat des (+)-1-[(3,4,5-Trimethoxy)-benzyloxymethyl]-1-phenyl-N,N-dimethyl-n-propylamin,
dadurch gekennzeichnet, daß
(+)-2-Dimethylamino-2-phenyl-n-butanol der nachstehenden Formel

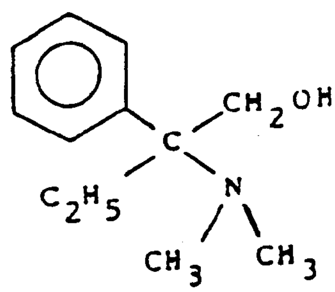

umgesetzt wird mit einem Halogenid des 3,4,5-Trimethoxybenzyls der nachstehenden Formel

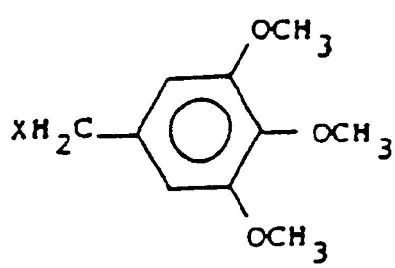

wobei X für Halogen steht,vorzugsweise für Chlor, Brom oder Iod und das Salz durch Salzbildung mit einer Säure gebildet wird.

2. Verfahren zur Herstellung von (+)-1-[(3,4,5-Trimethoxy)-benzyloxymethyl]-1-phenyl-N,N-dimethyl-n-propylamin der nachstehenden Formel

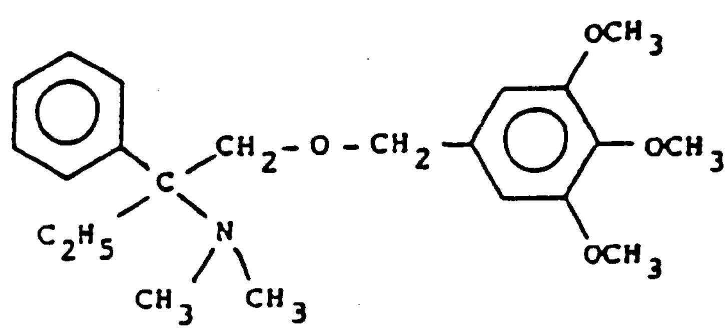

und seiner Additionssalze mit therapeutisch verträglichen Salzen, insbesondere des D-(-)Tartrat des (+)-1-[(3,4,5-Trimethoxy)-benzyloxymethyl]-1-phenyl-N,N-dimethyl-n-propylamin
gekennzeichnet durch die Verfahrensschritte:

- aus der Lösung des entsprechenden Propylracemat in einem Lösungsmittel wird durch Zugabe einer rechtsdrehenden organischen Säure "(+)-Säure" das in der Lösung unlösliche Salz "(+)-Säure-(-)-Propylamin" gebildet;
- dieses unlösliche Salz wird durch Filtrieren entfernt, um eine Lösung zu erhalten, die zugleich die (+)-Säure und das (+)-Enantiomere des n-Propylamin enthält;
- diese Lösung wird zur Trockene eingedampft, um das Lösungsmittel zu entfernen und um einen Rückstand zu erhalten;
- der Rückstand wird in Wasser aufgelöst und die erhaltene Lösung wird durch Zugabe einer Alkalihydroxid-, Erdalkalihydroxid oder Ammoniak-Lösung alkalisch gemacht bis zu einem pH-Wert in der Nähe von 10 oder darüber;
- das freigesetzte, rechtsdrehende n-Propylamin wird mit einem organischen Lösungsmittel extrahiert, und daraufhin wird dieses organische Extraktionslösungsmittel durch Eindampfen entfernt, um das gesuchte (+)-n-Propylamin zu erhalten.

3. Verfahren nach Anspruch 4,
dadurch gekennzeichnet, daß
zur Salzbildung mit dem Propylamin-Racemat in einem Lösungsmittel L-(+)-weinsäure zugesetzt wird.

4. Verfahren zur Herstellung eines in der Gastroenterologie brauchbaren Arzneimittels,
dadurch gekennzeichnet, daß
das nach den Ansprüchen 1 bis 3 hergestellte rechtsdrehende Amin oder eines seiner Salze, das/die eine lokale Wirkung auf die Opiatrezeptoren des Magen-Darm-Traktes - ohne merkliche zentrale Wirkung - und ferner eine späsmolytische Wirkung aufweist/aufweisen und ein pharmazeutisch verträglicher Trägerstoff vermischt werden.

**Patentansprüche für folgenden Vertragsstaat : GR**

1. (+)-1-[(3,4,5-Trimethoxy)-benzyloxymethyl]-1-phenyl-N,N-dimethyl-n-propylamin der nachstehenden Formel

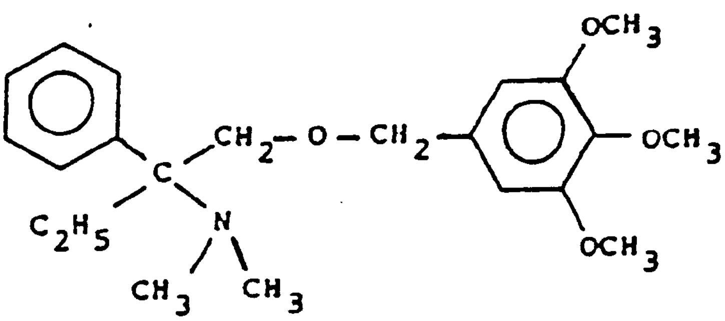

und dessen Additionssalze mit Säuren.

2. D-(-)-Tartrat des (+)-1-[(3,4,5-Trimethoxy)-benzyloxymethyl]-1-phenyl-N,N-dimethyl-n-propylamin.

3. Verfahren zur Herstellung des (+)-1-[(3,4,5-Trimethoxy)-benzyloxymethyl]-1-phenyl-N,N-dimethyl-n-propylamin,
dadurch gekennzeichnet, daß
(+)-2-Dimethylamino-2-phenyl-n-butanol der nachstehenden Formel

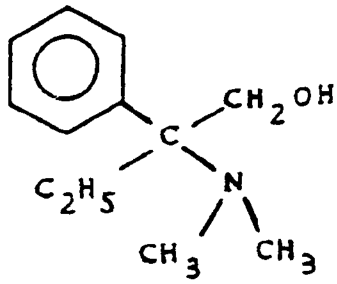

umgesetzt wird mit einem Halogenid des 3,4,5-Trimethoxybenzyl der nachstehenden Formel

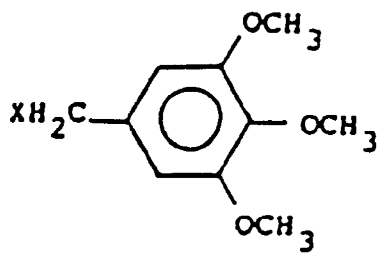

wobei X für Halogen steht, vorzugsweise für Chlor, Brom oder Iod.

**4.** Verfahren zur Herstellung von (+)-1-[(3,4,5-Trimethoxy)-benzyloxymethyl]-1-phenyl-N,N-dimethyl-n-propylamin,
gekennzeichnet durch die Verfahrensschritte:
- aus der Lösung des entsprechenden Propylracemat in einem Lösungsmittel wird durch Zugabe einer rechtsdrehenden organischen Säure "(+)-Säure" das in der Lösung unlösliche Salz "(+)-Säure-(-)-Propylamin" gebildet;
- dieses unlösliche Salz wird durch Filtrieren entfernt, um eine Lösung zu erhalten, die zugleich die (+)-Säure und das (+)-Enantiomere des n-Propylamin enthält;
- diese Lösung wird zur Trockene eingedampft, um das Lösungsmittel zu entfernen und um einen Rückstand zu erhalten;
- der Rückstand wird in Wasser aufgelöst und die erhaltene Lösung wird durch Zugabe einer Alkalihydroxid-, Erdalkalihydroxid oder Ammoniak-Lösung alkalisch gemacht bis zu einem pH-Wert in der Nähe von 10 oder darüber;
- das freigesetzte, rechtsdrehende n-Propylamin wird mit einem organischen Lösungsmittel extrahiert, und daraufhin wird dieses organische Extraktionslösungsmittel durch Eindampfen entfernt, um das gesuchte (+)-n-Propylamin zu erhalten.

**5.** Verfahren nach Anspruch 4,
dadurch gekennzeichnet, daß
zur Salzbildung mit dem Propylamin-Racemat in einem Lösungsmittel L-(+)-Weinsäure zugesetzt wird.

**6.** Verfahren nach Anspruch 3 oder 4,
dadurch gekennzeichnet, daß
zur Salzbildung des gesuchten (+)-n-Propylamin dessen Salz mit einer pharmazeutisch verträglichen Säure gebildet wird.

**7.** Verfahren zur Herstellung eines in der Gastroenterologie brauchbaren Arzneimittels,
dadurch gekennzeichnet, daß
das nach den Ansprüchen 1 bis 3 hergestellte rechtsdrehende Amin oder eines seiner Salze, das/die eine lokale Wirkung auf die Opiatrezeptoren des Magen-Darm-Traktes - ohne merkliche zentrale Wirkung - und ferner eine spasmolytische Wirkung aufweist/aufweisen mit einem pharmazeutisch verträglichen Trägerstoff vermischt wird.

**8.** Verwendung des Propylamin nach Anspruch 1 oder eines seiner therapeutisch verträglichen Salze zur

Herstellung eines Medikaments, das eine lokale Wirkung auf die Opiatrezeptoren des Magen-Darm-Traktes - ohne merkliche zentrale Wirkungen - und ferner eine spasmolytische Wirkung aufweist.

## Claims

### Claims for the following Contracting States : AT, BE, CH, DE, FR, GB, IT, LI, LU, NL, SE

1. (+)-1-[(3,1,5-trimethoxy)-benzyloxymethyl]-1-phenyl-N,N-dimethyl-n-propylamine of formula

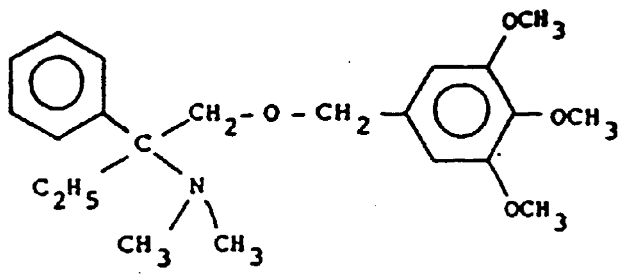

and its pharmaceutically acceptable acid addition salts.

2. (+)-1-[(3,4,5-Trimethoxy)-benzyloxymethyl]-1-phenyl-N,N-dimethyl-n-propylamine D-(-)-tartrate.

3. Process for preparing (+)-1-[(3,4,5-trimethoxy)-benzyloxymethyl]-1-phenyl-N,N-dimethyl-n-propylamine characterised in that it comprises reacting (+)-2-dimethylamino-2-phenyl-n-butanol of formula

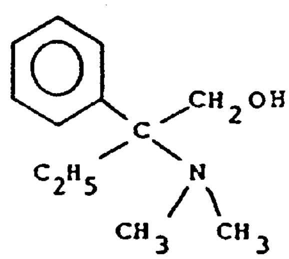

with a 3, 4, 5-trimethoxybenzyl halide of formula

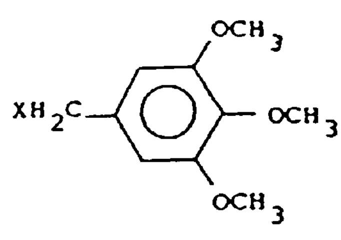

wherein X is a halogen, preferably chlorine, bromine or iodine.

4. Process for preparing (+)-1-[(3,4,5-trimethoxy)-benzyloxymethyl]-1-phenyl-N,N-dimethyl-n-propylamine characterised in that it comprises salifying the corresponding racemic propylamine, dissolved in a solvent, using a (+)-dextrorotatory organic acid to give an insoluble (+) acid/(-) propylamine salt, removing this insoluble salt by filtration in order to obtain a solution containing both (+)-acid and the (+)-enantiomer of the n-propylamine, evaporating the solvent of this solution to dryness in order to obtain a residue, dissolving the residue in,water and alkalizing the solution to a pH near or above 10, by adding an alkali metal, alkaline earth or ammonium hydroxide-solution, extracting the free dextrorotatory n-propylamine with an organic solvent, and then evaporating this extracted organic solvent in order to obtain the desired (+)-n-propylamine.

5. Process according to claim 4 which comprises salifying the racemic propylamine in a solvent, using L-(+)-tartaric acid.

6. Process according to claim 3 or claim 4,
characterised in that it comprises preparing a salt of the desired (+)-n-propylamine by salifying it, using a pharmaceutically acceptable acid.

7. Medicament for use in gastroenterology,
characterised in that it comprises the dextrorotatory amine or a salt thereof according to claims 1 and 2 having a local action on the opiate receptors of the gastrointestinal tract, substantially without central actions, and also having a spasmolytic action.

**Claims for the following Contracting State : ES**

1. Process for preparing (+)-1-[(3,4,5-trimethoxy)-benzyloxymethyl]-1-phenyl-N,N-dimethyl-n-propylamine of formula

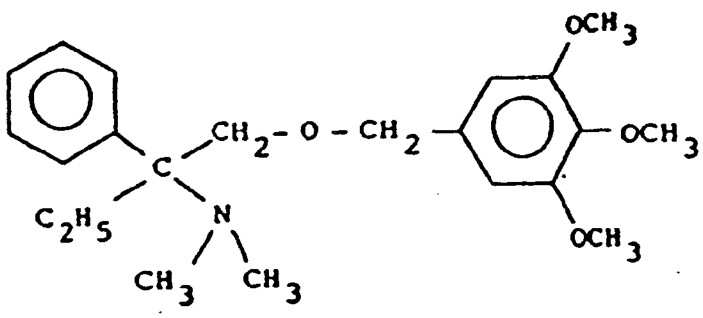

and its pharmaceutically acceptable acid addition salts, in particular (+)-1-[(3,4,5-Trimethoxy)-benzyloxymethyl]-1-pheny-N,N-dimethyl-n-propylamine D-(-)-tartrate characterised in that it comprises reacting (+)-2-dimethylamine-2-phenyl-n-butanol of formula

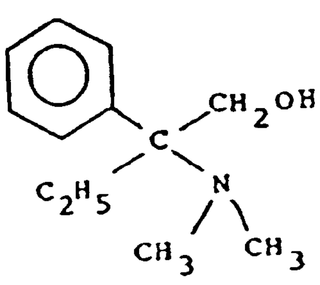

with a 3,4,5-trimethoxybenzyl halide of formula

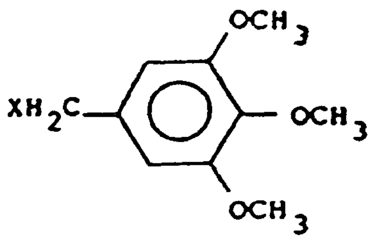

wherein X is a halogen, preferably chlorine, bromine or iodine, and for preparing a salt, salifying with an acid.

2. Process for preparing (+)-1-[(3,4,5-trimethoxy)-benzyloxymethyl)-1-phenyl-N,N-dimethyl-n-propylamine, in particular (+)-1-[(3,4,5,Trimethoxy)-benzyloxy-methyl]-1-pheny-N,N-dimethyl-n-propylamine D-(-)-tartrate characterised in that it comprises salifying the corresponding racemic propylamine, dissolved in a solvent, using a (+)dextrorotatory organic acid to give an insoluble (+) acid/(-) propylamine salt, removing this insoluble salt by filtration in order to obtain a solution containing both (+)-acid and the (+)-enantiomer of the n-propylamine, evaporating the solvent of this solution to dryness in order to obtain a residue, dissolving the residue in water and alkalizing the solution to a pH near or above 10, by adding an alkali metal, alkaline earth or ammonium hydroxide solution, extracting the free destrorotatory n-propylamine with an organic solvent, and then evaporating this extracted organic solvent in order to obtain the desired (+)-n-propylamine and, for preparing a salt, salifying it with an acid.

3. Process according to claim 2, which comprises salifying the racemic propylamine in a solvent, using L-(+)-tartaric acid.

4. Process for preparing a medicament for use in gastroenterology, characterised in that it comprises mixing the dextrorotatory amine or a salt thereof prepared according to the preceeding claims having a local action on the opiate receptors of the gastrointestinal tract, substantially without central actions, and also having a spasmolytic action, with a pharmaceutically acceptable carrier.

**Claims for the following Contracting State : GR**

1. (+)-1-[(3,1,5-trimethoxy)-benzyloxymethyl]-1-phenyl-N,N-dimethyl-n-propylamine of formula

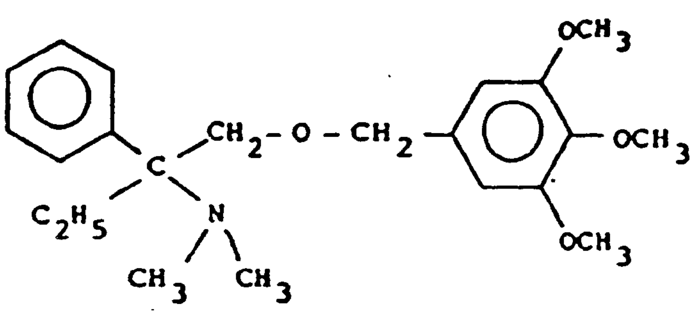

and its pharmaceutically acceptable acid addition salts.

2. (+)-1-[(3,4,5-Trimethoxy)-benzyloxymethyl]-1-phenyl-N,N-dimethyl-n-propylamine D-(-)-tartrate.

3. Process for preparing (+)-1-[(3,4,5-trimethoxy)-benzyloxymethyl]-1-phenyl-N,N-dimethyl-n-propylamine characterised in that it comprises reacting (+)-2-dimethylamino-2-phenyl-n-butanol of formula

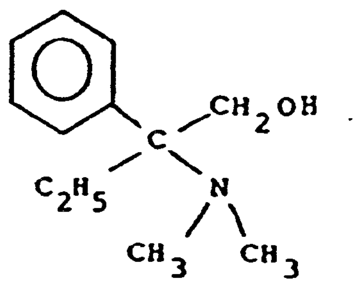

with a 3,4,5-trimethoxybenzyl halide of formula

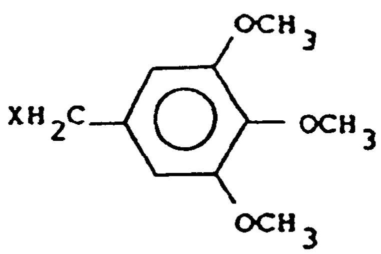

wherein X is a halogen, preferably chlorine, bromine or iodine.

4. Process for preparing (+)-1-[(3,4,5-trimethoxy)-benzyloxymethyl]-1-phenyl-N,N-dimethyl-n-propylamine characterised in that it comprises salifying the corresponding racemic propylamine, dissolved in a solvent, using a (+)-dextrorotatory organic acid to give an insoluble (+) acid/(-) propylamine salt, removing this insoluble salt by filtration in order to obtain a solution containing both (+)-acid and the (+)-enantiomer of the n-propylamine, evaporating the solvent of this solution to dryness in order to obtain a residue, dissolving the residue in water and alkalizing the solution to a pH near or above 10, by adding an alkali metal, alkaline earth or ammonium hydroxide solution, extracting the free dextrorotatory n-propylamine with an organic solvent, and then evaporating this extracted organic solvent in order to obtain the desired (+)-n-propylamine.

5. Process according to claim 4 which comprises salifying the racemic propylamine in a solvent, using L-(+)-tartaric acid.

6. Process according to claim 3 or claim 4, characterised in that it comprises preparing a salt of the desired (+)-n-propylamine by salifying it, using a pharmaceutically acceptable acid.

7. Process for preparing a medicament for use in gastroenterology, characterised in that it comprises mixing the dextrorotatory amine or a salt thereof according to claims 1 and 2 having a local action on the opiate receptors of the gastrointestinal tract, substantially without central actions, and also having a spasmolytic action, with a pharmaceutically acceptable carrier.

8. The use of the propylamine as defined in claim 1 or one of its therapeutically acceptable salts for preparing a medicament having a local action on the opiate receptors of the gastrointestinal tract, substantially without central actions, and also having a spasmolytic action.